# FASCICULE DE BREVET EUROPEEN

(11) **EP 4 000 029 B1**
(45) Date de publication et mention de la délivrance du brevet: **14.02.2024**
(21) Numéro de dépôt: 20734789.9
(22) Date de dépôt: 02.07.2020
(51) Int. Cl.: G06Q 20/18, G07F 17/18, G07F 17/00, A61F 15/00, G07F 11/00

(54) **DISTRIBUTEUR DE PRODUITS MENSTRUELS ET RECHARGES CORRESPONDANTES**
SPENDER FÜR MENSTRUATIONSPRODUKTE UND NACHFÜLLUNGEN FÜR MENSTRUATIONSPRODUKTE
MENSTRUAL PRODUCT DISPENSER AND REFILLS FOR MENSTRUAL PRODUCTS

(30) Priorité: 16.07.2019 FR 1908024; 13.08.2019 FR 1909191
(43) Date de publication de la demande: 25.05.2022
(73) Titulaire: Marguerite & Cie, 29740 Plobannalec-Lesconil (FR)
(72) Inventeur: LE NOANE, Gaële, 29740 PLOBANNALEC-LESCONIL (FR)
(74) Mandataire: Vidon Brevets & Stratégie
(86) Numéro de dépôt international: PCT/EP2020/068740
(87) Numéro de publication internationale: WO 2021/008891

(56) Documents cités:
- WO-A1-2019/074571
- DE-U1- 20 308 135
- DE-U1- 29 819 738
- US-A- 3 860 304
- US-A- 5 167 345
- US-A- 5 947 302
- US-A1- 2005 115 979
- US-A1- 2011 114 660

## Description

### Domaine technique

Le domaine de la présente technique est celui de la mise à disposition de produits menstruels, comme des tampons ou des serviettes hygiéniques. Plus particulièrement, la présente technique concerne des distributeurs permettant un libre accès à de tels produits menstruels.

### Art antérieur

Certaines entreprises, certains organismes publics ou certaines collectivités locales, territoriales ou nationales (facultés, collèges, lycées, cités universitaires ...) souhaitent offrir en libre accès, par exemple dans les toilettes de leurs locaux, des produits de santé et notamment des produits menstruels, pour des raisons de lutte contre les inégalités et contre la précarité des femmes. En effet, les produits menstruels peuvent représenter un coût très élevé pour certaines femmes en grandes difficultés financières.

Des distributions de ces produits, sous leur forme commercialisée (c'est-à-dire dans les emballages prévus pour être vendus) sont déjà mises en oeuvre, par exemple dans des lieux à vocation d'entraide et d'accompagnement, grâce à des collectes de produits préalablement effectuées.

Il existe aussi une solution de mise à disposition de produits menstruels sous la forme de distributeurs présentant par exemple un ou deux compartiments de stockage de produits, lesquels s'accumulent ensuite dans un compartiment ouvert permettant l'accès libre aux produits. Ce type de distributeurs présentent cependant au moins deux inconvénients importants, à savoir le non-respect de certaines règles d'hygiène indispensables pour ce type de produits et l'absence d'ergonomie et de praticité pour les personnes en charge de l'approvisionnement de ces distributeurs. En effet, ces distributeurs doivent être remplis, par leur partie supérieure, avec des produits « en vrac », ce qui n'est pas pratique. De plus, la présence d'un grand nombre de produits dans le compartiment ouvert, et donc un accès potentiel à plusieurs produits au moment d'en prendre un pour son propre usage posent des problèmes d'hygiène évidents, sans compter la manipulation de produits « en vrac » au moment de l'approvisionnement du distributeur.

Il est connu du document WO2019/074571 un distributeur de produits d'hygiène féminine dont la mise à disposition est activée par un capteur de mouvement.

Il est connu du document US2011/114660 un distributeur dans lequel une pile ou un rouleau de produits absorbants peut être inséré.

Il est par ailleurs connu du document US2005/115979 un rouleau ou pliage d'un ensemble de produits absorbants, qui peut être inséré directement dans un distributeur.

Il existe toutefois un besoin d'une solution de mise à disposition de produits menstruels qui soit ergonomique pour les utilisatrices ainsi que pour les personnes en charge de leur gestion, tout en respectant les contraintes d'hygiène liées à ce type de produits.

### Exposé de l'invention

La présente technique répond à ce besoin en proposant un distributeur de produits menstruels selon la revendication 1.

Ainsi, la présente technique propose une solution nouvelle et inventive de la mise à disposition de produits menstruels permettant non seulement une gestion optimisée grâce à un distributeur prévu pour recevoir une ou plusieurs recharges de ces produits menstruels mais aussi une hygiène renforcée.

Ainsi, le distributeur de la présente solution présente un ou plusieurs compartiments destinés à recevoir une ou plusieurs recharges comprenant par exemple chacune un type de produits menstruels (tampons, de tailles différentes, avec ou sans applicateur, serviettes hygiéniques de tailles différentes), facilitant ainsi l'approvisionnement du distributeur mais également le stockage des produits. En effet, le personnel en charge de l'approvisionnement d'un distributeur n'a pas à alimenter le distributeur directement en produits, disponibles par exemple « en vrac » dans un sac ou un carton, mais a simplement à insérer une ou plusieurs recharges, pleines et fermées, dans le distributeur, puis à ouvrir chaque recharge pour rendre disponibles les produits, comme décrit ci-après.

Par ailleurs, un tel distributeur permet une mise à disposition de plusieurs types de produits différents, grâce à plusieurs compartiments permettant chacun la réception d'une recharge de produits différents.

De plus, ce système de recharges permet de respecter les contraintes d'hygiène liées aux produits menstruels, tant au niveau de la gestion des distributeurs qu'au moment de leur utilisation.

Ainsi, l'utilisation de recharges, fermées, pour alimenter le distributeur, permet d'éviter toute manipulation directe des produits au moment de l'approvisionnement du distributeur.

De même, ces contraintes d'hygiène sont également respectées lors de la mise à disposition des produits menstruels, grâce à la forme spécifique du compartiment de mise à disposition et à l'ouverture prévue dans le compartiment de stockage, qui permettent de limiter le nombre de produits disponibles, en ne rendant accessible qu'un produit à la fois (ou plus exactement un seul produit de chaque type à la fois, si plusieurs types de produits sont distribués). De cette manière, une utilisatrice n'a accès qu'au produit qu'elle va utiliser, sans toucher un ou plusieurs autres produits destinés ultérieurement à une autre utilisatrice.

Selon l'invention, la face avant présente au moins une ouverture inférieure en regard dudit au moins un compartiment de mise à disposition, ladite ouverture présentant une forme et des dimensions adaptées pour la mise à disposition d'un seul produit menstruel par type dans ledit compartiment de mise à disposition.

Ainsi, la face avant présente une ouverture adaptée pour permettre la descente des produits de la charge vers le compartiment de mise à disposition de sorte à ce qu'un seul produit de chaque type soit accessible à la fois, grâce également à la forme inférieure des recharges comme décrit ci-après. De cette manière, les contraintes d'hygiène sont respectées, ainsi qu'un fonctionnement optimisé du distributeur en évitant tout blocage ou engorgement de produits dans les recharges.

Selon l'invention, l'ouverture ménagée dans la face avant présente au moins une encoche de préhension.

Ainsi, l'ouverture inférieure est prévue pour faciliter la préhension d'un produit dans le compartiment de mise à disposition, tout en empêchant l'accès à plusieurs produits d'un même type.

En effet, pour faciliter la prise d'un produit, une solution simple aurait été de prévoir une ouverture inférieure plus large. En revanche, une telle solution aurait favorisé la descente de plusieurs produits (par type) dans le compartiment de mise à disposition, contrevenant ainsi aux exigences d'hygiène déjà évoquées ci-dessus.

De plus, comme cela sera décrit ci-après, en relation avec les recharges de produits, cette encoche a une double fonction et permet également l'ouverture de la recharge une fois insérée dans le compartiment de stockage. En effet, une encoche similaire prédécoupée sur la recharge fermée se situe en regard de l'encoche de la face avant lorsque la recharge est insérée et un simple appui sur la recharge, via l'encoche de la face avant, permet de découper la recharge et d'en ouvrir la partie inférieure pour libérer les produits vers le compartiment de mise à disposition.

De préférence, l'encoche de préhension est centrée par rapport à la largeur du compartiment de stockage.

De plus, la taille de cette encoche, et plus particulièrement sa hauteur, peut être variable. Selon une première variante, elle présente une hauteur d'environ un centimètre permettant d'obtenir l'effet technique souhaité. Selon une deuxième variante, elle présente une hauteur pouvant aller jusqu'à dix centimètres, de manière à permettre en plus un éventuel déblocage des produits dans la recharge, tout en offrant une visibilité sur le stock de produits restants dans la recharge.

Selon une caractéristique particulière, le distributeur comprend au moins deux compartiments de stockage séparés par une paroi verticale sensiblement parallèle auxdites faces latérales, lesdits au moins deux compartiments de stockage recevant chacun une recharge de produits.

Ainsi, lorsque le distributeur comprend au moins deux compartiments de stockage, il est possible de n'insérer qu'une seule recharge qui sera non seulement guidée dans le distributeur mais également maintenue en position adéquate par la paroi verticale, y compris en l'absence d'une recharge dans le compartiment voisin.

En effet, sans cette paroi, la recharge isolée pourrait ne pas rester verticale dans le distributeur et donc empêcher la descente des produits vers le compartiment de mise à disposition.

Une telle paroi correspond par exemple à un simple rail de guidage plus ou moins profond, solidaire de la face arrière, ou de la face avant du distributeur. Une telle paroi peut également correspondre à une cloison pleine traversant le distributeur de la face arrière à la face avant.

Selon un aspect particulier du distributeur, sa base présente un angle supérieur à 90° avec ladite face arrière.

Ainsi, la forme de la base du distributeur, adaptée à celle de la base des recharges (comme décrit ci-après), permet une distribution optimale des produits, en offrant une pente d'un angle avantageusement choisi pour permettre la descente des produits au fur et à mesure de la prise des produits, par les utilisatrices, dans le compartiment de mise à disposition, sans blocage ni engorgement.

Selon un mode de réalisation particulier, le distributeur comprend également au moins un couvercle supérieur mobile présentant au moins une position fermée et une position ouverte pour l'introduction de ladite au moins une recharge de produits et les faces latérales sont sensiblement verticales et perpendiculaires à ladite face arrière et s'étendent dudit couvercle jusqu'à l'extrémité extérieure dudit compartiment de mise à disposition.

Ainsi, les parois latérales du distributeur présentent une forme particulière leur conférant un premier effet technique de renforcement de la rigidité du distributeur, en solidarisant les compartiments de stockage et de mise à disposition. En effet, du fait de son emplacement et de sa forme, le compartiment de mise à disposition constitue un point de fragilité au niveau de sa liaison avec le ou les compartiments de stockage, cette fragilité étant réduite voire supprimée grâce au renfort des parois latérales du distributeur.

De plus, cette forme spécifique prévue pour les parois latérales permet également au distributeur de ne pas présenter d'angles aigus au niveau du compartiment de mise à disposition qui constitue une excroissance dans la partie inférieure du distributeur. Ainsi, quelle que soit la forme du compartiment de mise à disposition, les parois latérales présentent une forme adaptée pour s'étendre de manière à englober de chaque côté le compartiment de mise à disposition.

Par exemple, le compartiment de mise à disposition présente une forme :
- en arc de cercle, ou
- en V, ou
- à fond plat avec un rebord, perpendiculaire au fond ou incliné par rapport au fond.

Ainsi, la forme du compartiment de mise à disposition peut dépendre par exemple de contraintes de fabrication et de matériau. Par exemple, une forme en arc de cercle (ou demi-gouttière) peut être difficile à réaliser dans certains matériaux ou selon certains procédés de fabrication : par exemple lorsque les autres parties du distributeur peuvent être fabriquées par pliage d'une matière telle que de la tôle fine, l'obtention d'une forme arrondie n'est pas possible par pliage, alors qu'une forme à fond plat avec un rebord peut être réalisable plus facilement.

Quelle que soit la forme du compartiment de mise à disposition, celui-ci doit pouvoir permettre l'accès à un produit de chacun des types de produits prévus dans le distributeur, ainsi que le bon maintien des produits en attendant qu'ils soient utilisés. Ainsi, la forme en gouttière doit être suffisamment profonde et le rebord d'un compartiment à fond plat doit être suffisamment haut pour retenir les produits, de chaque type.

De plus, selon un aspect particulier, le compartiment de mise à disposition présente au moins une paroi de séparation en regard de ladite au moins une paroi dudit compartiment de stockage.

Selon ce mode de réalisation, il est prévu une paroi de séparation au niveau du compartiment de mise à disposition, de manière à contenir un type de produit lorsqu'il descend de la recharge. En particulier, cette paroi est utile pour des produits de petite taille, comme les tampons sans applicateurs, qui risquent de se déplacer dans le compartiment de mise à disposition et de se mélanger avec les autres produits.

De plus, comme décrit ci-après, les recharges contenant ces petits produits ne présentent pas, contrairement à celles pour les produits de plus grande taille, d'encoches de rétention de produits. Les tampons sans applicateur sont donc plus susceptibles que les autres de bouger lors de leur descente dans le compartiment de mise à disposition et doivent donc être maintenus dans un espace dédié, grâce à cette paroi.

La présente technique concerne également une recharge de produits selon la revendication 5.

Le distributeur de produits menstruels est donc prévu pour recevoir une ou plusieurs recharges de produits, de forme sensiblement parallélépipédique avec une base « biseautée », lesquelles sont insérées pleines et fermées dans le distributeur. Elles présentent donc un prédécoupage permettant de les ouvrir, dans leur partie inférieure, une fois insérées dans le distributeur par la personne en charge de l'approvisionnement.

De plus, la forme « biseautée » de la base des recharges est avantageusement prévue pour faciliter la descente des produits dans la recharge, au fur et à mesure de la prise des produits, par des utilisatrices, dans le compartiment de mise à disposition du distributeur.

Par exemple, une telle recharge est réalisée en matière cartonnée ou en papier rigide.

Selon l'invention, la forme prédéterminée de prédécoupage comprend au moins une encoche destinée à se situer en regard d'une encoche similaire prévue dans ledit distributeur de produits menstruels lorsque ladite recharge est insérée dans ledit distributeur de produits menstruels.

L'ouverture de la recharge est donc facilitée par la présence d'une encoche dans le prédécoupage, coïncidant avec une encoche similaire dans la face avant du distributeur, plus exactement du compartiment de stockage des recharges. De cette manière, la personne en charge de l'approvisionnement du distributeur appuie sur la partie inférieure de la recharge accessible par l'ouverture inférieure prévue dans la face avant du distributeur, au niveau de l'encoche, de manière à pouvoir utiliser l'encoche de la recharge comme une languette pour terminer le découpage de la partie inférieure de la recharge et l'ouverture complète de la base de celle-ci.

De plus, comme déjà indiqué ci-dessus en relation avec le distributeur, cette encoche, une fois la recharge ouverte, facilite la prise du produit, par une utilisatrice, dans le compartiment de mise à disposition. En effet, pour des raisons hygiéniques, l'ouverture prévue pour rendre accessible les produits étant restreinte, de manière à ne rendre accessible qu'un seul produit par type de produit, il pourrait être difficile de prendre le produit sans cette encoche.

De préférence, l'encoche est centrée par rapport à la largeur de la recharge.

Comme pour les encoches prévues dans le compartiment de stockage, et tel que décrit précédemment, la taille de cette encoche de la recharge, et plus particulièrement sa hauteur, peut être variable. Selon une première variante, elle présente une hauteur d'environ un centimètre. Selon une deuxième variante, elle présente une hauteur pouvant aller jusqu'à dix centimètres, de manière à permettre en plus un éventuel déblocage des produits dans la recharge, tout en offrant une visibilité sur le stock de produits restants dans la recharge.

Selon un aspect particulier, la forme prédéterminée de prédécoupage comprend au moins une languette formant une excroissance.

Ainsi, une fois la recharge ouverte, elle présente une encoche centrale permettant la préhension du produit dans le compartiment de mise à disposition, et au moins une languette permettant de retenir les produits dans la recharge, afin de limiter à un produit, par type, accessible dans ce compartiment de mise à disposition.

Avantageusement, et particulièrement pour les produits de grande taille comme les tampons avec applicateur ou les serviettes hygiéniques, deux languettes sont prévues sur la face avant de la recharge, de part et d'autre de l'encoche centrale, de manière à assurer un bon maintien des produits dans la recharge une fois ouverte.

Il est à noter que les recharges contenant des petits produits (comme par exemple des tampons sans applicateur) ne présentent pas ces languettes, la taille ajustée de la recharge et du compartiment de stockage correspondant ainsi que la présence d'une paroi dans le compartiment de mise à disposition permettant de limiter le nombre de produits accessibles.

De plus, le prédécoupage s'étend également sur les deux arêtes latérales et l'arête inférieure des recharges de manière à pouvoir retirer complètement la matière prédécoupée et permettre ainsi l'accès aux produits qui descendent et sortent de la recharge.

### Présentation des figures

D'autres buts, caractéristiques et avantages de la technique proposée apparaîtront plus clairement à la lecture de la description suivante, donnée à titre de simple exemple illustratif, et non limitatif, en relation avec les figures, parmi lesquelles :
[Fig 1] présente un distributeur de produits menstruels, sans recharge, selon un mode de réalisation de la technique proposée ;
[Fig 2a] présente une vue de face du distributeur de produits menstruels de la figure 1, selon une première variante ;
[Fig 2b] présente une vue de face du distributeur de produits menstruels de la figure 1, selon une deuxième variante ;
[Fig 3] présente une vue en perspective du distributeur de produits menstruels de la figure 1, selon la première variante ;
[Fig 4a] présente une vue de côté du distributeur de produits menstruels de la figure 1, selon une première variante de réalisation du compartiment de mise à disposition ;
[Fig 4b] présente une vue de côté du distributeur de produits menstruels de la figure 1, selon une deuxième variante de réalisation du compartiment de mise à disposition ;
[Fig 5] présente une vue du dessus du distributeur de produits menstruels de la figure 1 ;
[Fig 6a] présente un distributeur de produits menstruels avec des recharges fermées, selon une première variante d'un mode de réalisation de la technique proposée ;
[Fig 6b] présente un distributeur de produits menstruels avec des recharges ouvertes, selon la première variante d'un mode de réalisation de la technique proposée ;
[Fig 6c] présente un distributeur de produits menstruels avec des recharges ouvertes, selon une deuxième variante d'un mode de réalisation de la technique proposée ;
[Fig 7a] présente un exemple de recharge pour distributeur de produits menstruels de petite taille, selon un mode de réalisation de la technique proposée ;
[Fig 7b] présente un exemple de recharge pour distributeur de produits menstruels de grande taille, selon une première variante d'un mode de réalisation de la technique proposée ;
[Fig 7c] présente un exemple de recharge pour distributeur de produits menstruels de grande taille, selon une deuxième variante d'un mode de réalisation de la technique proposée.

### Description détaillée de modes de réalisation de l'invention

Le principe général de la technique proposée repose sur un distributeur de produits menstruels comprenant au moins un compartiment de stockage de produits menstruels, de plusieurs types, contenus dans des recharges et un compartiment de mise à disposition des produits agencé pour qu'un seul produit de chaque type de soit accessible pour les utilisatrices.

Ainsi, l'approvisionnement en produits du distributeur selon la solution proposée est facilité grâce à l'utilisation de recharges fermées à insérer dans le distributeur, et les règles d'hygiène liées aux types de produits distribués sont respectées, au moment de l'approvisionnement du distributeur grâce aux recharges fermées et au moment de l'accès aux produits grâce à la limitation à un seul produit accessible en même temps.

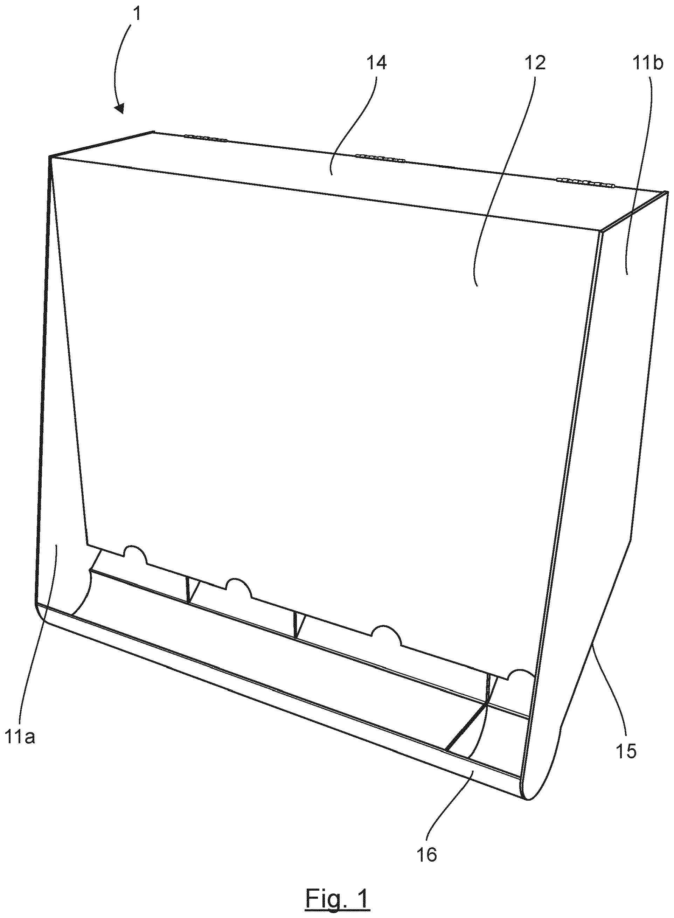

Comme illustré sur les Fig 1 à 5, un distributeur 1 selon un mode de réalisation de la technique proposée comprend donc une face arrière pour sa fixation sur un support, le plus couramment un mur dans des toilettes par exemple. Un tel distributeur 1 comprend également deux faces latérales 11a et 11b, et une face avant 12.

De plus, le distributeur 1 comprend au moins un compartiment de stockage des produits (13a, 13b, 13c, 13d), adapté pour recevoir une recharge de produits telle que décrite plus en détails ci-après. Pour permettre cette insertion de recharge(s) dans le distributeur 1, il est donc prévu un couvercle supérieur mobile 14 pouvant s'ouvrir pour approvisionner le distributeur 1 et se fermer pour empêcher l'accès aux recharges et donc aux produits.

Selon plusieurs variantes de réalisation, le couvercle 14 peut être verrouillé, ou simplement conçu de sorte à ce que les faces latérales 11a et 11b recouvrent le champ du couvercle 14, empêchant ainsi d'y accéder. Ceci constitue une forme de verrouillage facile à mettre en oeuvre, et les personnes en charge de l'approvisionnement du distributeur disposeront d'un aimant permettant d'ouvrir le couvercle pour insérer les recharges. D'autres formes de verrouillage peuvent bien sûr être mises en oeuvre, selon les besoins.

Le distributeur 1 comprend également une base 15, solidaire de la face arrière 10 et des faces latérales11a et 11b, pour le maintien inférieur des recharges dans le distributeur.

Selon une caractéristique particulière, la base 15 n'est pas perpendiculaire à la face arrière (le distributeur ne présente donc pas une forme parallélépipédique) mais elle forme un angle supérieur à 90° avec la face arrière, de manière à présenter une pente identique à celle formée par la base des recharges (voir description ci-après) afin de faciliter l'écoulement des produits vers la zone de distribution.

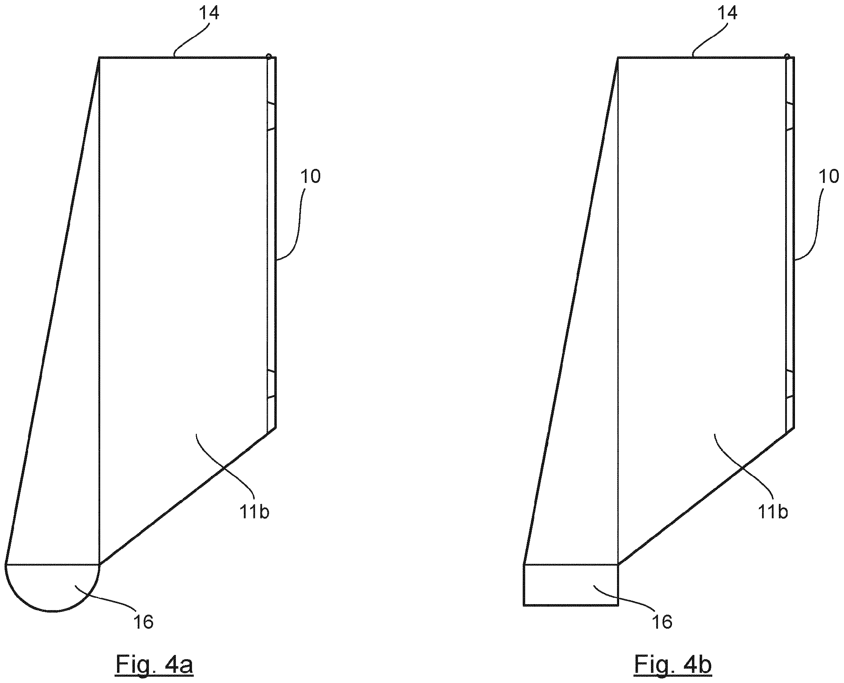

Cette zone de distribution correspond à un compartiment de mise à disposition 16, solidarisé, sur sa longueur, à la base 15 du distributeur. Ce compartiment de mise à disposition peut prendre plusieurs formes, comme par exemple une forme en arc de cercle (demi-gouttière) comme illustré en Fig 4a, en V, ou encore un fond plat avec un rebord pour retenir les produits, comme illustré en Fig 4b. Comme déjà indiqué, la forme du compartiment de mise à disposition peut être choisie selon des contraintes techniques de fabrication, de matériau, ou encore des souhaits esthétiques, à partir du moment où elle respecte les contraintes techniques liées au distributeur lui-même qui exigent qu'un seul produit, par type de produit, ne soit accessible dans le compartiment de mise à disposition 16.

De plus, ce compartiment de mise à disposition 16 ne doit pas être trop profond pour permettre une préhension aisée des produits par les utilisatrices, tout en assurant un bon maintien des produits qui ne doivent pas tomber du distributeur sous peine d'être inutilisables, toujours pour des raisons d'hygiène.

Une des particularités du distributeur 1 réside également dans l'ouverture 121 ménagée dans la face avant 12 pour permettre l'accessibilité à un produit par type de produit, lorsque le produit descend de la recharge pour se positionner dans le compartiment de mise à disposition 16. En effet, cette ouverture 121 ménagée dans la face avant présente une forme et des dimensions avantageusement déterminées pour obtenir cet effet technique de « libérer » un seul produit descendant d'une recharge tout en le rendant accessible, c'est-à-dire en lui permettant d'atteindre le compartiment de mise à disposition 16. L'ouverture 121 doit également permettre aux utilisatrices de prendre le produit dans le compartiment de mise à disposition 16.

Enfin, une difficulté supplémentaire pour la détermination de cette ouverture 121 réside dans la multiplicité des types de produits pouvant être distribués, et notamment dans la multiplicité de leurs formes et de leurs tailles. Ainsi, des tampons, avec ou sans applicateurs, de tailles différentes sont distribués à côté de serviettes hygiéniques, de tailles différentes également.

Enfin, comme décrit ci-après, cette ouverture 121 sert également à pouvoir ouvrir les recharges insérées dans les compartiments de stockage 13a à 13d, et doit permettre à la personne en charge de l'approvisionnement des distributeurs, d'accéder à la base de la recharge pour la découper, selon le prédécoupage prévu.

Pour ce faire, l'ouverture 121 n'est pas rectiligne mais présente au moins une encoche 122 dite de préhension, avantageusement prévue pour chaque compartiment de stockage du distributeur et de préférence centrée par rapport à la largeur de chaque compartiment. Cette encoche 122 permet également à la personne qui insère les recharges d'accéder à la base des recharges pour les ouvrir, comme décrit ci-après. La forme (par exemple en arc de cercle) et les dimensions de ces encoches de préhension (122a à 122d) sont donc avantageusement définies pour permettre d'obtenir ces différents effets techniques : ouverture des recharges et aide à la préhension d'un produit dans le compartiment de mise à disposition.

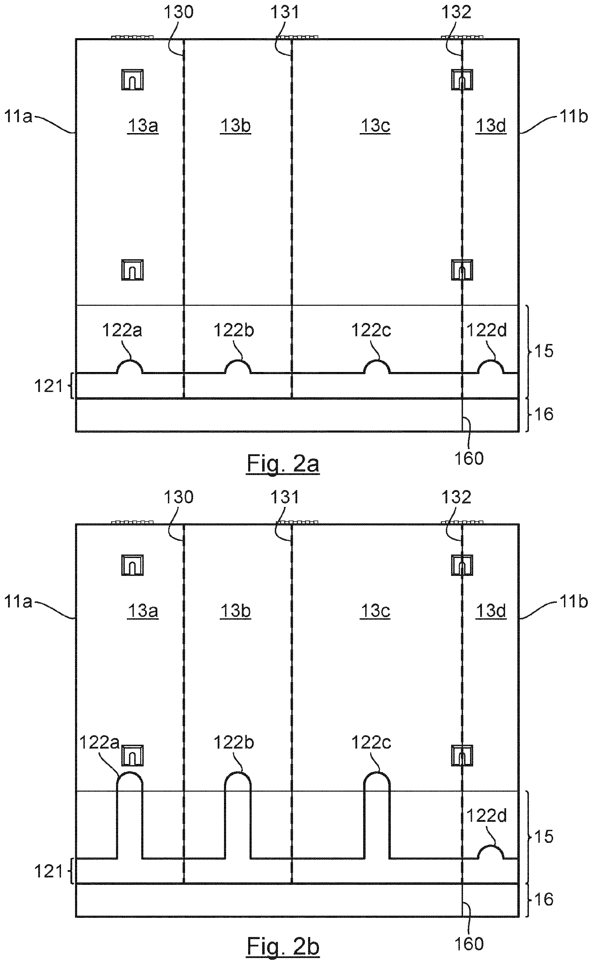

Par exemple, selon une première variante illustrée en Fig 2a, les encoches 122a à 122d présentent des dimensions sensiblement identiques, et notamment une hauteur d'environ 1 centimètre.

Selon une deuxième variante illustrée en Fig 2b, les encoches 122a à 122c présentent des dimensions sensiblement identiques, et notamment une hauteur pouvant aller jusqu'à dix centimètres, de manière à permettre un éventuel déblocage des produits dans la recharge, tout en permettant une visibilité sur le stock de produits restants dans la recharge. Ces encoches 122a à 122c correspondent donc plutôt à des fentes verticales. L'encoche 122d présente cependant une taille plus petite, et notamment une hauteur d'environ 1 centimètre, afin d'empêcher une éventuelle sortie des produits (de petites tailles) de la recharge.

Comme détaillé ci-après avec des exemples de dimensions possibles pour la réalisation d'un tel distributeur, l'ouverture 121 doit être très précisément définie pour respecter toutes ces contraintes et permettre d'obtenir les effets techniques recherchés.

Comme déjà indiqué précédemment, le distributeur 1 est avantageusement prévu pour offrir plusieurs types de produits menstruels différents, grâce à plusieurs compartiments de stockage 13a ...13i. Selon les exemples illustrés, quatre compartiments sont prévus, recevant quatre recharges contenant quarte types de produits différents. Il est bien sûr possible de prévoir plus ou moins de compartiments de stockage, selon les besoins, les caractéristiques du distributeur restant les mêmes dès qu'il y a au moins deux compartiments.

En effet, dès que le distributeur 1 comporte au moins deux compartiments de stockage, il est prévu que ces compartiments soient séparés par une paroi verticale 130 ... 13i, sensiblement parallèle aux faces latérales du distributeur 1. Selon les exemples illustrés, les quatre compartiments de stockage 13 à 13d sont respectivement séparées par des parois 130, 131 et 132. Ces parois 130, 131 et 132 permettent non seulement de guider les recharges pendant leur insertion, mais également de les stabiliser dans leur compartiment de stockage respectif, une fois insérée, même en l'absence d'une recharge dans le compartiment de stockage voisin.

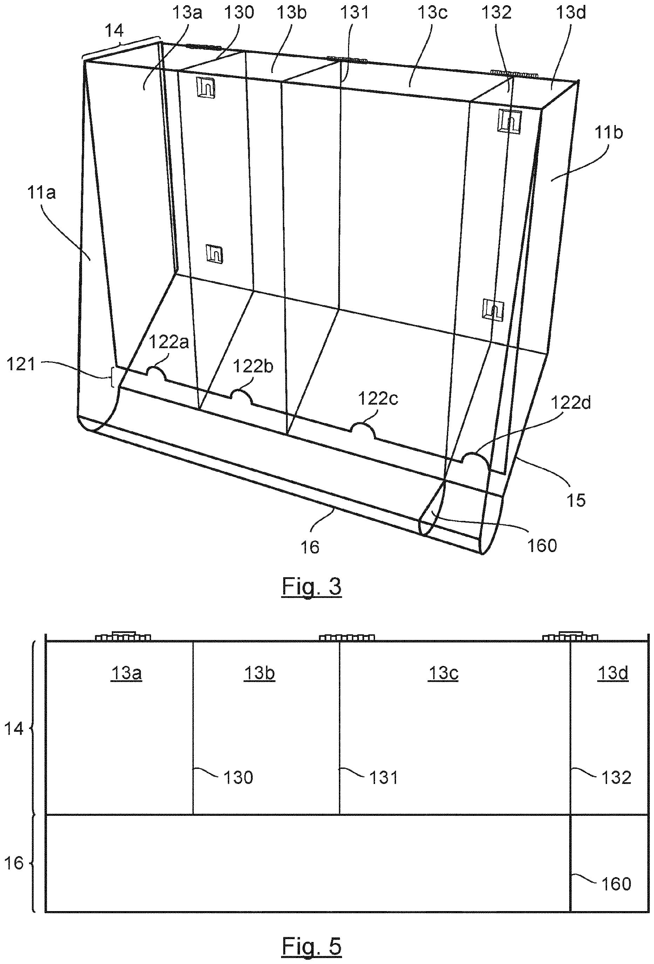

Selon une première deuxième variante de réalisation, illustrée par exemple en Fig 3 et en Fig 5, une paroi est formée par une cloison s'étendant de la face arrière à la face avant du distributeur, sur tout ou partie de la hauteur du compartiment de stockage.

Selon une deuxième variante de réalisation, non illustrée, une paroi est formée par un simple rail, plus ou moins large, sur tout ou partie de la hauteur du compartiment de stockage, solidaire de la face arrière ou de la face avant du distributeur.

Toute autre mode de réalisation de telles parois est envisageable dès lors qu'elles permettent d'obtenir l'effet technique souhaité, à savoir le guidage et le maintien des recharges dans leur compartiment de stockage dédié.

On décrit maintenant un autre aspect du distributeur 1, en relation avec les Fig 3 et 4a et 4b notamment, relatif à la forme des faces latérales 11a et 11b. En effet, selon un mode de réalisation de la technique proposée, les faces latérales 11a et 11b, sensiblement verticales et perpendiculaires à la face arrière, s'étendent au-delà de la face avant 12 dans leurs parties inférieures pour englober le compartiment de mise à disposition 16. Cette caractéristique permet d'une part de renforcer la rigidité du distributeur, en réduisant fortement, voire en annulant, le point de fragilité que pourrait constituer le compartiment de mise à disposition 16 (du fait de sa forme et de son unique zone de solidarisation avec le reste du distributeur) et d'autre part d'éviter tout angle aigu à l'extérieur du distributeur. Enfin, ce prolongement des parois latérales permet également d'offrir des rebords de chaque côté du compartiment de mise à disposition 16, d'un même tenant donc d'une mise en oeuvre aisée à la fabrication et solide.

Enfin, le compartiment de mise à disposition 16 présente, selon une mise en oeuvre particulière, au moins une paroi de séparation 160, en regard d'une séparation entre deux compartiments de stockage, par exemple, comme illustré sur les Fig 2a, 2b, 3 et 5, en regard de la paroi 132 séparant les compartiments de stockage 13c et 13d. Cette paroi de séparation 160 permet notamment au produit descendant de la recharge insérée dans ce compartiment de stockage 13d d'être maintenu dans le compartiment de mise à disposition 16, sans se déplacer et donc sans se mélanger par exemple avec le produit descendant de la recharge insérée dans le compartiment de stockage 13c. avantageusement, cette paroi est utile lorsque les produits sont de petite taille, comme des tampons sans applicateur.

Avant de décrire plus en détails d'autre aspect de la présente solution, relatif aux recharges de produits menstruels destinées à être insérées dans un distributeur tel que décrit ci-dessus, on présente ci-après les dimensions d'un distributeur selon un mode de réalisation de l'invention présente, adaptées à la distribution de quatre types de produits menstruels, via quatre compartiments de stockage :
- 430 millimètres de largeur totale,
- 351,5 millimètres de hauteur totale, jusqu'à l'extrémité extérieure de la base 15 (sans la profondeur du compartiment de mise à disposition 16),
- 104,25 millimètres de largeur pour le compartiment de stockage 13a,
- 103,5 millimètres de largeur pour le compartiment de stockage 13b,
- 163,5 millimètres de largeur pour le compartiment de stockage 13c,
- 54,25 millimètres de largeur pour le compartiment de stockage 13d,
- 25 millimètres de largeur pour chaque encoche de préhension 122,
- 25 millimètres d'ouverture 121 de la face avant 12,
- 115 millimètres de profondeur des compartiments de stockage 13a à 13d,
- 188 millimètres de profondeur maximale, y inclus la profondeur des compartiments de stockage 13a à 13d et la largeur du compartiment de mise à disposition 16,
- 259 millimètres de hauteur de la face arrière 10,
- 68 millimètres de largeur du compartiment de mise à disposition 16,
- 34 millimètres de profondeur du compartiment de mise à disposition 16.

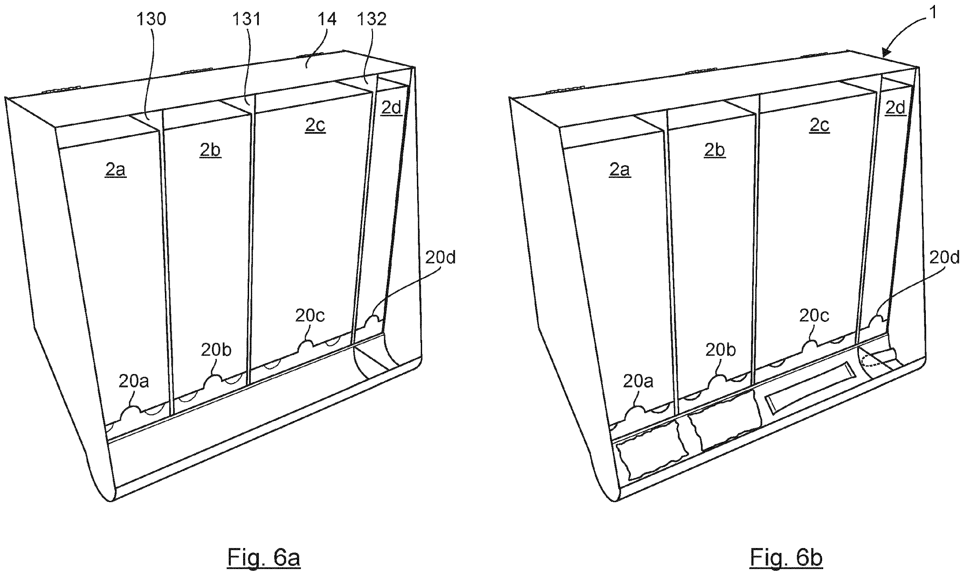

Les Fig 6a et 6b illustrent à présent un distributeur 1 selon une première variante d'un mode de réalisation décrit ci-dessus et dans lequel quatre recharges de produits 2a à 2d sont insérées, respectivement dans quatre compartiments de stockage 13a à 13d, séparés respectivement par des parois 130, 131 et 132, dans cet exemple.

Sur la Fig 6a, les recharges 2a à 2d sont fermées, telles qu'elles sont insérées par la personne en charge de l'approvisionnement du distributeur 1, alors que sur la Fig 6b, les recharges 2a à 2d sont ouvertes, la zone en blanc entre la partie inférieure de la face avant et le compartiment de mise à disposition 16 correspondant à l'intérieur des recharges, les produits étant également illustrés dans le compartiment de mise à disposition 16.

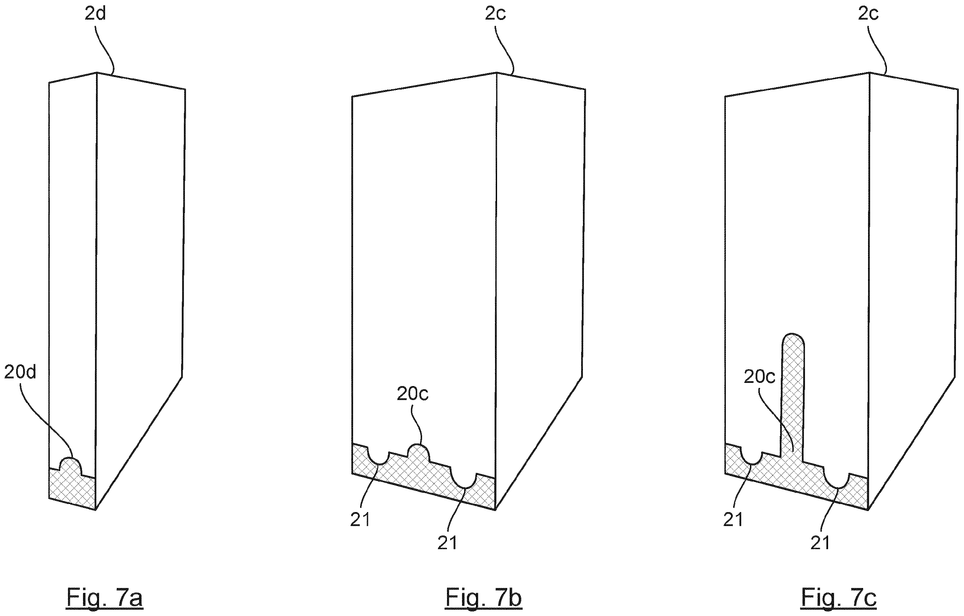

Deux exemples de recharges de produits sont illustrés en Fig 7a, 7b et 7c, pour deux types de produits comme par exemple respectivement des tampons sans applicateur, recharge étroite illustrée en Fig 7a et destinée à être insérée dans le compartiment de stockage 13d, et des tampons sans applicateur, recharge plus large illustrée en Fig 7b et 7c, selon deux variantes, et destinée à être insérée dans le compartiment de stockage 13c.

Comme pour la base du distributeur, une recharge de produits menstruels selon un mode de réalisation de la technique proposée comprend une partie supérieure sensiblement parallélépipédique, avec cependant une face avant de longueur supérieure à une face arrière et une base présentant un angle supérieur à 90° avec la face arrière, de sorte à ce que les compartiments de stockage du distributeur épousent la forme de chaque recharge. Cette base inclinée permet en effet de maitriser la descente des produits dans la recharge, au fur et à mesure de leur utilisation, tout en évitant tout blocage ou engorgement et en limitant à un seul produit accessible dans le compartiment de mise à disposition.

Par exemple, l'angle formé entre la base et la face arrière (pour une recharge ou pour le distributeur) se situe avantageusement aux alentours de 130 degrés.

De plus, la face avant de chaque recharge est prédécoupée selon une forme prédéterminée en partie inférieure pour permettre d'une part son ouverture lors de son installation dans le distributeur et également la mise à disposition des produits qu'elle contient, au fur et à mesure de leur utilisation.

En effet, afin de faciliter l'approvisionnement du distributeur et d'éviter toute manipulation directe des produits menstruels, chaque recharge est insérée fermée et la prédécoupe permet de faciliter son ouverture.

Ainsi, la prédécoupe comprend notamment une encoche (20a à 20d selon ce mode de réalisation), située de manière centrée par rapport à la largeur de la recharge et destinée à se trouver en regard de l'encoche 122a... 122d prévue sur la face avant du distributeur, tel que décrit précédemment. C'est sur cette encoche 20a à 20d que la personne en charge de l'approvisionnement du distributeur appuie, via l'encoche 122a... 122d prévue sur la face avant du distributeur, pour amorcer le découpage de la partie inférieure de la recharge, en vue de son ouverture totale. Une fois cette encoche 20a à 20d découpée, elle peut servir de languette pour tirer sur la partie inférieure de la recharge et ainsi découper de part et d'autre de l'encoche, y compris les arêtes latérales et l'arête inférieure, afin de détacher complètement la partie inférieure de la face avant de la recharge.

Selon une première variante illustrée en Fig 6a et Fig 6b, les encoches 20a à 20d présentent des dimensions sensiblement identiques, et notamment une hauteur d'environ 1 centimètre, afin de coïncider avec les encoches correspondantes prévues sur les recharges.

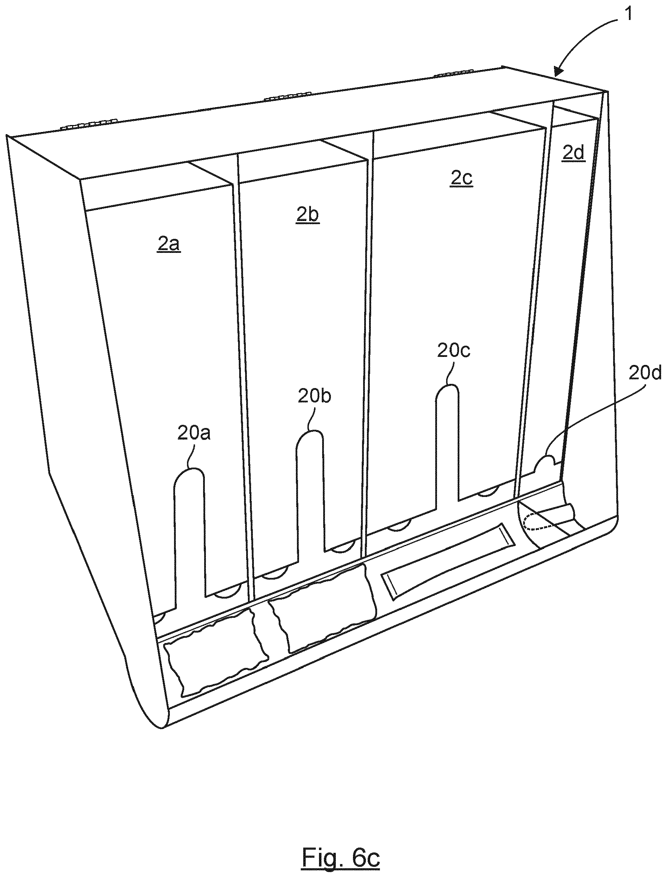

Selon une deuxième variante illustrée en Fig 6c, les encoches 20a à 20c présentent des dimensions sensiblement identiques, et notamment une hauteur pouvant aller jusqu'à dix centimètres, de manière à permettre un éventuel déblocage des produits dans la recharge, tout en permettant une visibilité sur le stock de produits restants dans la recharge. Ces encoches 20a à 20c correspondent donc plutôt à des fentes verticales. L'encoche 20d présente cependant une taille plus petite, et notamment une hauteur d'environ 1 centimètre, afin d'empêcher une éventuelle sortie des produits (de petites tailles) de la recharge.

Le résultat obtenu, c'est-à-dire des recharges ouvertes une fois insérées dans le distributeur, est par exemple illustré sur la Fig 6b.

Par ailleurs, afin d'optimiser l'effet technique souhaité consistant à n'avoir accès qu'à un produit de chaque type dans le compartiment de mise à disposition 16 du distributeur, la forme prédécoupée de la partie inférieure de chaque recharge comprend également au moins une languette 21 formant une excroissance de la recharge lorsqu'elle est ouverte, comme illustré sur la Fig 7b. Cette languette 21 est destinée en effet à retenir les produits dans la recharge, à l'exception du produit prévu pour être accessible dans le compartiment de mise à disposition. Par exemple, et comme illustré en Fig 7b, une recharge comprend deux languettes 21, situées de part et d'autre de l'encoche 20, notamment pour les recharges contenant des produits de grande taille, comme les tampons avec applicateurs ou les serviettes. Ainsi, dans l'exemple illustré en Fig 6a, 6b et 7a, les recharges 2a, 2b et 2c présentent deux languettes 21 et une encoche 20A, 20b et 20c, alors que la recharge 2d ne présente qu'une encoche 20d du fait de la petite taille des produits qu'elle contient. Ainsi, la taille de l'ouverture prévue dans la face avant du distributeur et ces languettes prévus dans la partie inférieure des recharges permettent d'obtenir l'effet technique souhaité.

Par ailleurs, sur les Fig 7a à 7c, la partie « découpable » de la recharge est hachurée. On peut ainsi notamment bien visualiser les encoches 20d et 20c, et en particulier les deux variantes de réalisation de l'encoche 20c, respectivement de taille sensiblement identique (hauteur d'environ 1 centimètre ) à l'encoche 20d comme illustré sur la Fig 7b, et de taille plus grande (notamment une hauteur pouvant aller jusqu'à dix centimètres) comme illustré sur la Fig 7c. Comme déjà décrit, cette plus grande taille de l'encoche, qui s'apparente à une fente verticale, permet un éventuel déblocage des produits dans la recharge, tout en permettant une visibilité sur le stock de produits restants dans la recharge.

La présente technique permet donc, grâce à un distributeur de produits menstruels et des recharges contenant les produits destinées à y être insérées, de proposer une solution de distribution de produits menstruels de plusieurs types, en respectant les contraintes d'hygiénique liées à ces produits, de façon ergonomique à la fois pour les utilisatrices et pour les personnes en charge d'approvisionner les distributeurs.

## Revendications

1. Distributeur de produits menstruels (1) comprenant une face arrière (10) destinée à la fixation dudit distributeur sur un support vertical, deux faces latérales (11a, 11b) et une face avant (12), **caractérisé en ce qu'**il comprend :
- au moins un compartiment de stockage (13a, 13b, 13c, 13d) adapté pour la réception d'au moins une recharge fermée comprenant une pluralité de produits menstruels d'un même type, ladite au moins une recharge comprenant une partie supérieure sensiblement parallélépipédique, une face avant de longueur supérieure à une face arrière et une base présentant un angle supérieur à 90° avec ladite face arrière,
ledit au moins un compartiment de stockage étant adapté pour épouser la forme de ladite au moins une recharge,
- au moins un compartiment de mise à disposition (16) d'au moins un desdits produits menstruels, et **en ce que** ladite face avant dudit distributeur présente au moins une ouverture inférieure (121) en regard dudit au moins un compartiment de mise à disposition (16),
ladite ouverture (121) présentant une forme et des dimensions adaptées pour la mise à disposition d'un seul produit menstruel par type dans ledit compartiment de mise à disposition, et
ladite ouverture présentant au moins une encoche de préhension (122, 122a, 122b, 122c, 122d) adaptée à être située en regard d'au moins une encoche (20a, 20b, 20c, 20d) d'une forme prédéterminée prédécoupée dans la face avant de ladite au moins une recharge, en sa partie inférieure, pour son ouverture et la mise à disposition des produits qu'elle contient, lorsque ladite au moins une recharge est insérée dans ledit distributeur.

2. Distributeur de produits menstruels selon la revendication 1, **caractérisé en ce qu'**il comprend au moins deux compartiments de stockage (13a, 13b, 13c, 13d) séparés par une paroi verticale (130, 131, 132) sensiblement parallèle auxdites faces latérales (11a, 11b), lesdits au moins deux compartiments de stockage étant chacun destinés à recevoir une recharge de produits.

3. Distributeur de produits menstruels selon l'une quelconque des revendications 1 et 2, **caractérisé en ce que** ledit distributeur comprend également une base (15) présentant un angle supérieur à 90° avec ladite face arrière (10) dudit distributeur.

4. Distributeur de produits menstruels selon l'une quelconque des revendications 1 à 3, **caractérisé en ce qu'**il comprend au moins un couvercle supérieur mobile (14) présentant au moins une position fermée et une position ouverte pour l'introduction de ladite au moins une recharge de produits, et **en ce que** lesdites faces latérales (11a, 11b) sont sensiblement verticales et perpendiculaires à ladite face arrière (10) et s'étendent dudit couvercle (14) jusqu'à l'extrémité extérieure dudit compartiment de mise à disposition (16).

5. Recharge fermée de produits (2a, 2b, 2c, 2d) comprenant une pluralité de produits menstruels d'un même type adaptée à être insérée dans un distributeur de produits menstruels selon la revendication 1, **caractérisée en ce que** ladite recharge comprend une partie supérieure sensiblement parallélépipédique, une face avant de longueur supérieure à une face arrière et une base présentant un angle supérieur à 90° avec ladite face arrière,
et **en ce que** ladite face avant est prédécoupée selon une forme prédéterminée en partie inférieure pour son ouverture et la mise à disposition des produits qu'elle contient,
ladite forme prédéterminée de prédécoupage comprenant au moins une encoche (20a, 20b, 20c, 20d) destinée à se situer en regard de ladite au moins une encoche de préhension (122, 122a, 122b, 122c, 122d) prévue dans ledit distributeur de produits menstruels lorsque ladite recharge est insérée dans ledit distributeur de produits menstruels.

6. Recharge fermée de produits selon la revendication 5 **caractérisé en ce que** ladite forme prédéterminée de prédécoupage comprend au moins une languette (21) formant une excroissance.

7. Distributeur selon la revendication 1 équipé d'au moins une recharge fermée selon la revendication 5.

## Patentansprüche

1. Spender für Menstruationsprodukte (1), umfassend eine Rückseite (10), die für die Befestigung des Spenders auf einer vertikalen Stütze bestimmt ist, zwei Seitenflächen (11a, 11b) und eine Vorderseite (12), **dadurch gekennzeichnet, dass** er umfasst:
- mindestens ein Lagerabteil (13a, 13b, 13c, 13d), das für die Aufnahme mindestens einer geschlossenen Nachfüllung, umfassend eine Vielzahl von Menstruationsprodukten desselben Typs geeignet ist, eine Vorderseite mit einer größeren Länge als eine Rückseite und eine Grundfläche, die einen Winkel von mehr als 90° mit der Rückseite bildet,
wobei das mindestens eine Lagerabteil geeignet ist, sich an die Form der mindestens einen Nachfüllung anzupassen,
- mindestens ein Spenderabteil (16), für mindestens eines der Menstruationsprodukte, und dass die Vorderseite des Spenders mindestens eine untere Öffnung (121) gegenüber dem mindestens einen Spenderabteil (16) aufweist, wobei die Öffnung (121) eine Form und Abmessungen aufweist, die für das Spenden eines einzigen Menstruationsproduktes pro Typ in dem Spenderabteil geeignet sind, und wobei die Öffnung mindestens eine Greifkerbe (122, 122a, 122b, 122c, 122d) aufweist, die geeignet ist, gegenüber mindestens einer Kerbe (20a, 20b, 20c, 20d) mit einer vorbestimmten Form angeordnet zu sein, die in der Vorderseite der mindestens einen Nachfüllung in ihrem unteren Teil vorausgeschnitten ist, um sie zu öffnen und die Produkte, die sie enthält zu spenden, wenn die mindestens eine Nachfüllung in den Spender eingefügt wird.

2. Spender für Menstruationsprodukte nach Anspruch 1, **dadurch gekennzeichnet, dass** er mindestens zwei Lagerabteile (13a, 13b, 13c, 13d) umfasst, die durch eine vertikale Wand (130, 131, 132), die im Wesentlichen zu den Seitenflächen (11a, 11b) parallel ist, getrennt sind, wobei die mindestens zwei Lagerabteile jeweils dazu bestimmt sind, eine Nachfüllung von Produkten aufzunehmen.

3. Spender für Menstruationsprodukte nach einem der Ansprüche 1 und 2, **dadurch gekennzeichnet, dass** der Spender auch eine Grundfläche (15) umfasst, die einen Winkel von mehr als 90° mit der Rückseite (10) des Spenders bildet.

4. Spender für Menstruationsprodukte nach einem der Ansprüche 1 bis 3, **dadurch gekennzeichnet, dass** er mindestens einen beweglichen oberen Deckel (14) umfasst, der mindestens eine geschlossene Position und eine offene Position zum Einführen der mindestens einen Produktnachfüllung aufweist, und dass die Seitenflächen (11a, 11b) im Wesentlichen vertikal und senkrecht auf die Rückseite (10) sind und sich vom Deckel (14) bis zum äußeren Ende des Spenderabteils (16) erstrecken.

5. Geschlossene Produktnachfüllung (2a, 2b, 2c, 2d), umfassend eine Vielzahl von Menstruationsprodukten eines selben Typs, die geeignet ist, in einen Spender für Menstruationsprodukte nach Anspruch 1 eingeführt zu werden, **dadurch gekennzeichnet, dass** die Nachfüllung einen im Wesentlichen parallelflachen oberen Teil, eine Vorderseite mit einer größeren Länge als eine Rückseite und eine Grundfläche aufweist, die einen Winkel von mehr als 90° mit der Rückseite bildet, und dass die Vorderseite nach einer vorbestimmten Form im unteren Teil vorausgeschnitten ist, um geöffnet zu werden und die Produkte, die sie enthält, zu spenden,
wobei die vorbestimmten vorausgeschnittene Form, umfassend mindestens eine Kerbe (20a, 20b, 20c, 20d), die dazu bestimmt ist, gegenüber der mindestens einen Greifkerbe (122, 122a, 122b, 122c, 122d), die in dem Spender für Menstruationsprodukte vorgesehen ist, angeordnet zu sein, wenn die Nachfüllung in den Spender für Menstruationsprodukte eingefügt wird.

6. Geschlossene Produktnachfüllung nach Anspruch 5, **dadurch gekennzeichnet, dass** die vorbestimmte vorausgeschnittene Form mindestens eine Lasche (21) umfasst, die eine Ausstülpung bildet.

7. Spender nach Anspruch 1, der mit mindestens einer geschlossenen Nachfüllung nach Anspruch 5 ausgestattet ist.

## Claims

1. Dispenser of menstrual products (1) comprising a rear face (10) intended for the fastening of said dispenser onto a vertical support, two lateral faces (11a, 11b) and a front face (12), **characterised in that** it comprises:
- at least one storage compartment (13a, 13b, 13c, 13d) adapted for the reception of at least one closed refill comprising a plurality of menstrual products of the same type, said at least one refill comprising a substantially parallelepipedic upper part, a front face having a length greater than a rear face and a base having an angle of more than 90° with said rear face,
said at least one storage compartment being adapted to conform to the shape of said at least one refill,
- at least one compartment for making available (16) at least one of said menstrual products,
and **in that** said front face of said dispenser has at least one lower opening (121) facing said at least one compartment for making available (16),
said opening (121) having a shape and dimensions adapted for making available a single menstrual product per type in said compartment for making available, and
said opening having at least one gripping notch (122, 122a, 122b, 122c, 122d) adapted to be located facing at least one notch (20a, 20b, 20c, 20d) having a predetermined shape pre-cut out in the front face of said at least one refill, in its lower part, for its opening and making available the products that it contains, when said at least one refill is inserted into said dispenser.

2. Dispenser of menstrual products according to claim 1, **characterised in that** it comprises at least two storage compartments (13a, 13b, 13c, 13d) separated by a vertical wall (130, 131, 132) substantially parallel to said lateral faces (11a, 11b), said at least two storage compartments each being intended to receive a refill of products.

3. Dispenser of menstrual products according to any one of claims 1 and 2, **characterised in that** said dispenser also comprises a base (15) having an angle of more than 90° with said rear face (10) of said dispenser.

4. Dispenser of menstrual products according to any one of claims 1 to 3, **characterised in that** it comprises at least one mobile upper cover (14) having at least a closed position and an open position for the insertion of said at least one refill of products, and **in that** said lateral faces (11a, 11b) are substantially vertical and perpendicular to said rear face (10) and extend from said cover (14) to the outer end of said compartment for making available (16).

5. Closed refill of products (2a, 2b, 2c, 2d) comprising a plurality of menstrual products of the same type adapted to be inserted into a dispenser of menstrual products according to claim 1, **characterised in that** said refill comprises a substantially parallelepipedic upper part, a front face having a length greater than a rear face and a base having an angle of more than 90° with said rear face,
and **in that** said front face is pre-cut according to a predetermined shape in the lower part for its opening and making available the products that it contains,
said predetermined pre-cutting shape comprising at least one notch (20a, 20b, 20c, 20d) intended to be located facing said at least one gripping notch (122, 122a, 122b, 122c, 122d) provided in said dispenser of menstrual products when said refill is inserted into said dispenser of menstrual products.

6. Closed refill of products according to claim 5 **characterised in that** said predetermined pre-cutting shape comprises at least one tab (21) forming a protrusion.

7. Dispenser according to claim 1 equipped with at least one closed refill according to claim 5.
